# EUROPÄISCHE PATENTANMELDUNG

(11) **EP 4 653 539 A1**
(43) Veröffentlichungstag der Anmeldung: **26.11.2025**
(21) Anmeldenummer: 24177931.3
(22) Anmeldetag: 24.05.2024
(51) Int. Cl.: C12P 17/04, C12N 9/04

(54) **VERFAHREN ZUR HERSTELLUNG VON 2,5-DIFORMYLFURAN**

(71) Anmelder: Annikki GmbH, 8074 Raaba-Grambach (AT); Treemera GmbH, 83064 Raubling (DE)
(72) Erfinder: Staunig, Nicole, 8074 Raaba-Grambach (AT)
(74) Vertreter: Schwarz & Partner Patentanwälte GmbH

(57) **Zusammenfassung**

Verfahren zur Herstellung von 2,5-Diformylfuran, indem 5-(Hydroxymethyl)furfural, welches in einer einphasigen wässerigen Lösung gelöst vorliegt, mit einer Oxidase *in vitro* zu 2,5-Diformylfuran oxidiert wird, welches und aus der wässerigen Lösung abgetrennt wird, dadurch gekennzeichnet, dass das Verfahren bei einer Temperatur zwischen 20 °C und 40 °C ausgeführt wird und dass die einphasige wässerige Lösung zu Beginn der Oxidation mindestens 35 g/l 5-(Hydroxymethyl)furfural gelöst enthält, sodass das sich bildende 2,5-Diformylfuran unmittelbar aus der wässerigen Lösung ausfällt. (Figur 1)

## Beschreibung

Die Erfindung betrifft ein Verfahren zur Herstellung von 2,5-Diformylfuran aus 5-(Hydroxymethyl)furfural.

### Hintergrund der Erfindung

2,5-Diformylfuran (DFF; 2,5-Furandicarbaldehyd) ist ein heteroaromatisches Dialdehyd, das aufgrund seiner reaktiven Aldehyd-Funktionen beispielsweise als bifunktionelles Quervernetzungsreagenz für Proteine (als biobasierte Alternative für Glutaraldehyd) verwendet werden kann (Danielli et al., 2022).

DFF kann des Weiteren auch zur Herstellung von Polymeren, Tensiden, fluoreszierenden Materialien, Pharmazeutika sowie Aerogelen verwendet werden (Dai, 2021; Acosta et al., 2021; Derflinger et al., 2021).

Der Ausgangsstoff für die Herstellung von DFF ist 5-(Hydroxymethyl)furfural (HMF), das beispielsweise aus Cellulose (und damit aus nachwachsenden Rohstoffen) gewonnen werden kann. Durch die enzymatische oder chemische Hydrolyse entsteht aus Cellulose D-Glucose, welche in weiterer Folge enzymatisch oder chemisch zu D-Fructose isomerisiert wird. Mittels Dehydratisierung (Abspaltung von insgesamt drei H₂O-Molekülen) gelangt man von D-Fructose zu HMF. Gängige Systeme zur Dehydratisierung von Fructose sind zum einen Mineralsäuren wie H₂SO₄ oder HCl und zum anderen (Brønsted- oder Lewis-)saure Feststoffkatalysatoren (Cong et al., 2021; US 9617234 B1).

Der Oxidationsschritt (Umwandlung der Hydroxymethyl-Gruppe in eine Formyl-Gruppe) kann entweder chemisch-katalytisch oder biokatalytisch bewerkstelligt werden.

Für die heterogen-katalytische Oxidation von HMF zu DFF mit Sauerstoff können unterschiedliche Übergangsmetallkatalysatoren (z.B. Vanadium-, Mangan-, Molybdän-, Ruthenium-Oxide) eingesetzt werden (Dai, 2021). Als Alternative zu den im Allgemeinen nicht nachhaltigen und zum Teil teuren Metallkatalysatoren existiert ein Verfahren, bei dem HMF zuerst nur in Gegenwart von DMSO durch Dehydratisierung aus D-Fructose hergestellt wird (Amarasekara et al., 2008), welches in weiterer Folge durch Zugabe von katalytischen Mengen an NaBr oder HBr zur Reaktionsmischung zu DFF oxidiert wird. Die Nachteile an diesem Verfahren sind die hohen Reaktionstemperaturen (150 °C) und die Bildung von schwer zu entfernenden Nebenprodukten (aus DMSO) (Laugel et al., 2014).

Halliday et al. (2003) beschreiben ebenfalls ein zweistufiges Verfahren zur Herstellung von DFF aus D-Fructose. Die Dehydratisierung wurde mittels Kationentauscher-Harzes (AG 50W-X8) in DMSO durchgeführt (25,5 h bei 80 °C). Danach wurde das Harz abfiltriert und das HMF in Lösung wurde mit Sauerstoff in Gegenwart von 5 mol% V₂O₅ zu DFF oxidiert (17 h bei 150 °C). Zur Aufarbeitung wurde die Reaktionslösung mit Dichlormethan verdünnt, abfiltriert, mit Wasser gewaschen und durch Kieselgel filtriert. Das nach der Verdampfung des Lösungsmittels erhaltene Rohprodukt enthielt noch Verunreinigungen wie Dimethylsulfon (Me₂SO₂) und Dimethylsulfid (Me₂S), die aus DMSO gebildet wurden. Das Rohprodukt wurde daher zuerst mittels Vakuum-Sublimation und danach mittels Soxhlet-Extraktion (mit Cyclohexan als Extraktionsmittel) und gleichzeitiger Filtration durch Kieselgel gereinigt. Auf diese Weise konnte DFF mit einer Reinheit von >99 % und einer Ausbeute von 42% gewonnen werden.

Eine detaillierte Übersicht zur chemisch-katalytischen Herstellung von DFF aus HMF oder auch direkt aus D-Fructose sowie weiteren Kohlenhydraten findet sich im Artikel von Dai (2021).

Zur Herstellung von DFF sind auch biokatalytische Verfahren beschrieben, die sich durch mildere Reaktionsbedingungen (weniger kosten- und energieintensiv), die Erzeugung von weniger Abfall (wie Lösungsmittel oder Nebenprodukte) sowie bioabbaubare Katalysatoren (Zellen oder Enzyme) auszeichnen.

So beschreiben Acosta et al. (2021) den Pilz *Fusarium culmorum* EAN 51 zur Herstellung von DFF aus HMF. So konnten 92% des Startmaterials (50 mM HMF) in 31 h zu DFF oxidiert werden. Das gebildete DFF wurde durch Extraktion mit Ethylacetat aus dem Reaktionsmedium abgetrennt.

Li et al. (2023) nutzen *Escherichia coli*-Zellen mit exprimierter Galactose-Oxidase (GOase), einem Kupferhaltiges Enzym, um aus Brotresten oder aus D-Fructose hergestelltes HMF (Dehydratisierung im stark eutektischen Lösungsmittel Betain-Milchsäure) zu DFF zu oxidieren, wobei 0,631 g DFF/g D-Fructose sowie 0,323 g DFF/g Brot erhalten wurden.

Auch enzymatische (*in vitro*) Verfahren zur Oxidation von HMF zu DFF sind bekannt. Enzyme, die die Oxidation von HMF zu DFF katalysieren, sind Alkohol-Oxidasen (AO; z.B. von *Candida boidinii, Hansenula* sp. oder *Pichia pastoris*), Aryl-Alkohol-Oxidasen sowie die kommerziell erhältliche Pyranose-2-Oxidase von *Cariolus* sp. (US 8183020 B2; Qin et al., 2015).

Ein weiteres Enzym zur Oxidation von HMF zu DFF ist die HMF-Oxidase (HMFO; EC 1.1.3.47), die in Bakterien, Pilzen, aber auch in der Honigbiene (*Apis mellifera*) zu finden ist. Tjallinks et al. (2023) nutzten die "beeHMFO" zur Oxidation von 50 mM HMF zu DFF (91% in 29 h), wobei das Produkt der Überoxidation (5-Formyl-2-furancarbonsäure = FFA) als Nebenprodukt (7%) gefunden wurde.

Die GOase aus *Dactylium dendroides* kann beispielsweise 30 mM HMF innerhalb von 96 h in Gegenwart von Meerrettich-Peroxidase (HRP; zur Aktivierung der GOase) sowie Katalase (zur Zersetzung des entstehenden Wasserstoffperoxids) zu 92% zu DFF oxidieren (Qin et al., 2015).

Cajnko et al. (2020) testeten eine Reihe von kommerziell erhältlichen Enzymen (AO aus *Pichia pastoris*; GOase aus *Dactylium dendroides*; Katalase aus *Aspergillus niger*; Laccase aus *Trametes versicolor*; eine pilzliche Lignin-Peroxidase sowie HRP) auf 10 mM HMF und konnten nur für die AO und GOase die Bildung von DFF (Umsatz: 25,6% für AO und 5,1% für GO) beobachten. Im Fall der AO wurde auch FFA (3,1% Umsatz) gefunden. Durch Kombination der AO und der Katalase konnte der Umsatz von 25,6% auf 97,5% (in 72 h) gesteigert werden. Der Umsatz der Oxidation mittels GOase konnte durch die Zugabe von Katalase und HRP erhöht werden, jedoch verwendeten die Autoren Natrium-Phosphat-Puffer (pH 7) für die enzymatischen Umsetzungen (Cajnko et al., 2020). Wie Qin et al. (2015) beobachteten, sind die Umsätze der (Kupfer-haltigen) GOase in Phosphat-Puffern niedriger als in anderen Medien (wie Natrium-Acetat-Puffer oder deionisiertem Wasser), was auf die Bildung von schwerlöslichem Cu₃(PO₄)₂ zurückzuführen sein könnte.

McKenna et al. (2017) verwendeten eine Variante der Galactose-Oxidase (GOase M₃₋₅) zur Oxidation von HMF zu DFF. So konnten 100 mM HMF mit einem System bestehend aus GOase M₃₋₅, HRP und Katalase in 1 h zu 80% zu DFF oxidiert werden. Die Autoren beobachteten die höchsten Umsätze in Phosphat-Puffern, obwohl der Wildtyp des Enzyms in Phosphat-Puffern schlechtere Umsätze liefert (siehe oben). Das Enzymsystem ist auch in US 10344307 B2 beschrieben.

Milić et al. (2024) verwendeten den auf einem Epoxy-Carrier immobilisierten GOase-Wildtyp zur Oxidation von 50 mM HMF zu DFF (maximale Ausbeute 11,3% in 72 h; bei Austausch des Biokatalysators alle 24 h) in einer 50/50 (v/v)-Mischung aus Ethylacetat (EtOAc) und 0,1 M Natriumphosphat-Puffer (pH 7,4). Die Verwendung einer organischen Phase (EtOAc) hat zwei Gründe: 1. Erhöhung der Löslichkeit von HMF und DFF, 2. Verhinderung der Adsorption von DFF und HMF an der Oberfläche des Carriers. In reinem EtOAc wurde bei Einsatz der freien GOase keine Oxidation von HMF beobachtet.

EtOAc wird aufgrund seines niedrigen Siedepunkts (77 °C) durch die zur Oxidation notwendige Zufuhr von Sauerstoff unweigerlich aus dem Reaktionsgefäß ausgeblasen. Birmingham et al. verwendeten daher ein höher siedendes Lösungsmittel (Diethylcarbonat, bp = 126 °C) als Kosolvent bei ihrem Prozess. Mit der GOase M_{7-2A} kann HMF (100 g/l; 793 mM), das teilweise noch Verunreinigungen vom Produktionsprozess enthält, innerhalb von 6 h in Gegenwart von HRP und Katalase sowie Diethylcarbonat zu DFF (Umsatz 96%) oxidiert werden. Bei einer Substratkonzentration von 150 g/l (entspricht 1,19 M) können innerhalb von 6 h nur mehr 62% Umsatz erzielt werden. In einem weiteren Versuch (siehe Supplementary Table 7 in Birmingham et al.) wurde der Einfluss verschiedener Lösungsmittelmischungen (einphasig oder zweiphasig) im Vergleich zu Puffer (Natriumphosphat-Puffer, pH 7,4) auf die Umsetzung von 250 mM HMF getestet. Dabei konnten die höchsten Umsätze (75% und 71%) mit 40% EtOAc (zweiphasig) bzw. 10% DMSO (einphasig) als Kosolvent erzielt werden, in reinem Puffer konnten immerhin 50% des HMFs zu DFF umgesetzt werden. Um die Prozesskosten zu senken, schlagen die Autoren die Ersetzung der HRP durch einen adäquaten chemischen oder elektrochemischen Aktivator vor (Birmingham et al., 2021). Das zweiphasige Verfahren wurde auch in EP 3444355 A1 beschrieben.

In der Patent-Anmeldung EP 3444354 A1 wird ein Verfahren zur Abtrennung von DFF aus einer wässerigen Lösung mittels Abkühlung beschrieben. Dieses vorbekannte Verfahren ist sehr aufwendig: In einem ersten Reaktionsgefäß (Gefäß A) wurde 250 mM (31,5 g/l) vorgereinigtes HMF mittels GOase, Meerrettich-Peroxidase und Katalase in 800 ml Kalium-Phosphat-Puffer bei 20 °C oxidiert. In einem zweiten Reaktionsgefäß (Gefäß B) wurden 200 ml Kalium-Phosphat-Puffer (pH 7) auf 2 °C heruntergekühlt. Die Reaktionsmischung aus Gefäß A, welche gelöstes HMF und DFF enthielt, wurde kontinuierlich mit einer Rate von 37 ml/min in Gefäß B gepumpt, wo das DFF bedingt durch Abkühlung ausfiel. Der wässerige Überstand wurde zur weiteren Reaktion wieder in Gefäß A zurückgepumpt. Nach einer Reaktionszeit von 6 h fiel DFF als Feststoff aus. Zusätzliches DFF konnte durch Eindampfen der wässerigen Reaktionslösung gewonnen werden, wobei das auf diese Art gewonnene DFF noch Verunreinigungen des Startmaterials enthielt.

Hier setzt nun die Aufgabe der vorliegenden Erfindung an und setzt sich zum Ziel, ein biokatalytisches Verfahren zur Herstellung von 2,5-Diformylfuran (DFF) zur Verfügung zu stellen, welches einfacher durchgeführt werden kann.

### Detaillierte Beschreibung der Erfindung

Diese Aufgabe wird erfindungsgemäß gelöst, indem 5-(Hydroxymethyl)furfural, welches in einer einphasigen wässerigen Lösung gelöst vorliegt, *in vitro* mit einer Oxidase zu 2,5-Diformylfuran oxidiert wird, welches aus der wässerigen Lösung abgetrennt wird, und ist dadurch gekennzeichnet, dass das Verfahren bei einer Temperatur zwischen 20 °C und 40 °C ausgeführt wird und dass die einphasige wässerige Lösung zu Beginn der Oxidation mindestens 35 g/l 5-(Hydroxymethyl)furfural (entspricht ca. 278 mM) gelöst enthält, sodass das sich bildende 2,5-Diformylfuran aus der wässerigen Lösung ausfällt.

Unter den genannten Bedingungen fällt das sich bei der Oxidation bildende 2,5-Diformylfuran somit unmittelbar als Feststoff aus.

Das erfindungsgemäße Verfahren ist somit sehr einfach durchführbar: es kommt ohne ein aufwendiges Kühlen der Reaktionslösung aus und es ist auch kein organisches Kosolvent erforderlich. Darüber hinaus hat sich überraschenderweise gezeigt, dass das nach dem erfindungsgemäßen Verfahren hergestellte 2,5-Diformylfuran aus der wässerigen Lösung in hoher Reinheit ausfällt.

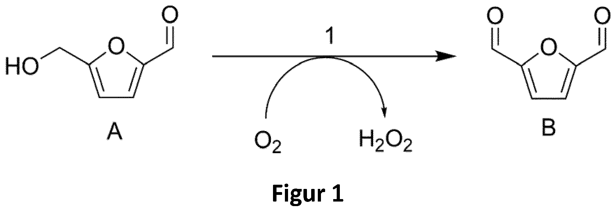

Das erfindungsgemäße Verfahren ist in der beiliegenden Figur 1 schematisch dargestellt, wobei die Bezeichnung A für 5-(Hydroxymethyl)furfural (HMF), B für 2,5-Diformylfuran (DFF) und 1 für Oxidase steht.

In einer bevorzugten Variante enthält die wässerige Lösung das 5-(Hydroxymethyl)furfural zwischen 50 g/l und 250 g/l, insbesondere zwischen 150 g/l und 250 g/l gelöst.

Weiters bevorzugt ist, wenn die wässerige Lösung Katalase enthält, um das sich bei der Oxidation bildende Wasserstoffperoxid zu zerstören.

Das erfindungsgemäße Verfahren kommt gänzlich ohne organische Kosolventien aus, und das bringt einen weiteren Vorteil: die geringe Löslichkeit des Produkts in einem wässrigen Medium erlaubt die leichte Abtrennung mittels Filtration.

Der besonders bevorzugte Temperaturbereich liegt zwischen 25 und 40 °C.

Der besonders bevorzugte pH-Bereich der Reaktion liegt zwischen pH 6 und pH 9.

Das Oxidationsmittel für die Umsetzung ist Sauerstoff, der entweder in Form von Pressluft oder in Reinform in das Reaktionsgefäß eingebracht wird. Zusätzlich kann die Sauerstoff-Konzentration in der Reaktionsmischung durch Anlegen eines Überdrucks im Reaktionsgefäß erhöht werden.

Das Enzym zur Oxidation von HMF zu DFF ist bevorzugt eine Oxidase, wobei die Hydroxymethylfurfural-Oxidase (HMFO; EC 1.1.3.47) besonders bevorzugt ist.

Die Oxidation wird bevorzugt *in vitro* durchgeführt.

Das Enzym liegt in einer Suspension, im Homogenat oder im Lysat der entsprechenden, das Enzym bildenden Zellen vor, wobei ein Lysat besonders bevorzugt sind.

Suspension bedeutet im Sinne der vorliegenden Beschreibung und Patentansprüche eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von KohlenstoffQuellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme & Herstellung der Lysate* für Details).

In einer weiteren Variante kann das Enzym auch mit einem wasserlöslichen Polymer wie Polyethylenglycol am N-Terminus modifiziert, in oder auf einer festen Matrix immobilisiert oder Teil eines Fusionsproteins sein.

In einer weiteren Variante kann das Enzym in Pulverform, in lyophilisierter oder sprühgetrockneter Form vorliegen.

Das hier beschriebene Verfahren erlaubt die kontinuierliche Produktion von DFF. Der ausgefallene Feststoff kann hierbei kontinuierlich mittels eines geeigneten Separators von der wässerigen Reaktionslösung abgetrennt werden, welche wieder in das Reaktionsgefäß rückgeführt wird. Zusätzlich kann auch wieder neues 5-(Hydroxymethyl)furfural der Reaktionslösung zugeführt werden.

Mit den nachfolgenden Beispielen werden bevorzugte Ausführungsformen der Erfindung noch näher beschrieben.

### Materialien

5-(Hydroxymethyl)furfural (HMF) wurde von Biosynth, 2,5-Diformylfuran (2,5-Furandicarbaldehyd; DFF) wurde von Sigma-Aldrich, Kaliumdihydrogenphosphat, di-Kaliumhydrogenphosphat sowie Natriumdodecylsulfat (SDS) wurden von Carl Roth, Acetonitril wurde von PanReac AppliChem (ITW Reagents) und Triethanolamin wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia* coli-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E. coli*-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen, mit Puffer versetzt (z.B. Triethanolamin (TEA) - HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymtypen und Spenderorganismen für das verwendete Enzym.**

| **Enzymtyp** | **katalysierte Reaktion(en)** | **Spenderorganismus** | **Literatur** |
|---|---|---|---|
| HMF-Oxidase* (HMFO) | HMF → DFF | *Pseudomonas nitroreducens* | (NCBI Protein Database: WP_024766380.1) |
| | DFF → FFA ** | | |

| | | | |
|---|---|---|---|
| Anmerkung *: In der NCBI Protein Database wird die HMF-Oxidase aus *P. nitroreducens* als Glucose-Methanol-Cholin (GMC) Oxidoreduktase klassifiziert, welche als Überfamilie auch die HMF-Oxidasen beinhaltet (Viñambres et al., 2020). Anmerkung **: Die verwendete HMF-Oxidase katalysiert auch die Reaktion von DFF zu dem "Überoxidationsprodukt" 5-Formylfurancarbonsäure (FFA). | | | |

### Analytische Methoden

### High Performance Liquid Chromatography (HPLC)

Zur Quantifizierung von HMF und DFF wurde HPLC (High Performance Liquid Chromatography) verwendet. Detektion erfolgt mittels eines UV-Detektors. Zur Messung wird eine Phenomenex Rezex ROA-Organic Acid H+ (8%) Säule mit entsprechender Vorsäule verwendet und mit 1 mM Schwefelsäure isokratisch eluiert.

Mit den nachfolgenden Beispielen werden bevorzugte Varianten des erfindungsgemäßen Verfahrens noch näher beschrieben. Das in diesen Beispielen eingesetzte Lysat wurde nach dem oben beschriebenen Verfahren hergestellt.

### Beispiel 1

### Oxidation von HMF zu DFF

Die Reaktion wurde in einem Labfors 5 Tisch-Bioreaktor (Infors AG) durchgeführt. Als Gefäß wurde ein Glasreaktor (Volumen 3,4 I) mit aufgesetztem Rührwerk, pH-Elektrode und O₂-Sensor verwendet. Die pH-Kontrolle erfolgte durch die Zudosierung von 5M NaOH oder 1M H₂SO₄.

Zu Beginn wurden 62 g HMF (ca. 88% Reinheit), 257,2 ml deionisiertes Wasser sowie 100 ml eines 500 mM Kaliumphosphat-Puffers (pH 7) im Reaktor vorgelegt und unter Rühren auf 20 °C gebracht. Dabei wurde eine klare braune Lösung (finale HMF-Konzentration 125 g/l; 993 mM) erhalten.

Zum Start der Reaktion wurden 80 ml HMFO-Lysat zugesetzt. Die Sauerstoff-Zufuhr (über einen Sparger) wurde auf 0,05 l/min gestellt.

Nach einiger Zeit beginnt sich die Lösung einzutrüben (Bildung von schlechter löslichem DFF).

Während des Betriebs wurden laufend Proben von der Reaktorlösung genommen, die folgendermaßen analysiert wurden: 50 µl der Reaktorlösung wurden mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Nach 30 h Laufzeit wurden 50 ml der Reaktionsmischung entnommen. Der ausgefallene Feststoff wurde direkt abfiltriert, zweimal mit je 10 ml H₂O gewaschen und über Nacht im Vakuumtrockenschrank bei 40 °C getrocknet.

Auf diese Weise konnten 4,2 g DFF als bräunlicher Feststoff (Verteilung der Analyten: 98,8% DFF; 1,2% HMF) isoliert werden. Es konnte keine FFA im Feststoff detektiert werden.

### Beispiel 2

### Oxidation von HMF zu DFF - Produktausfällung durch Abkühlung

Die Reaktion wurde in einem Labfors 5 Tisch-Bioreaktor (Infors AG) durchgeführt. Als Gefäß wurde ein Glasreaktor (Volumen 3,4 I) mit aufgesetztem Rührwerk, pH-Elektrode und O₂-Sensor verwendet. Die pH-Kontrolle erfolgte durch die Zudosierung von 5M NaOH oder 1M H₂SO₄.

Zu Beginn wurden 31 g HMF (ca. 88% Reinheit), 288,2 ml deionisiertes Wasser sowie 100 ml eines 500 mM Kaliumphosphat-Puffers (pH 7) im Reaktor vorgelegt und unter Rühren auf 20 °C gebracht. Dabei wurde eine klare braune Lösung (finale HMF-Konzentration 64 g/l; 508 mM) erhalten.

Zum Start der Reaktion wurden 80 ml HMFO-Lysat zugesetzt. Die Sauerstoff-Zufuhr (über einen Sparger) wurde auf 0,05 l/min gestellt.

Nach einiger Zeit beginnt sich die Lösung einzutrüben (Bildung von schlechter löslichem DFF).

Während des Betriebs wurden laufend Proben von der Reaktorlösung genommen, die folgendermaßen analysiert wurden: 50 µl der Reaktorlösung wurden mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Nach 4 h Laufzeit wurden 50 ml der Reaktionsmischung entnommen und im Kühlschrank bei 2 °C inkubiert. Die weiteren Aufarbeitungsschritte wurden analog zu Beispiel 1 (Abfiltrieren und Trocknung) durchgeführt.

Auf diese Weise konnten 1,6 g DFF als bräunlicher Feststoff (Verteilung der Analyten: 97,4% DFF; 1,6% HMF; 1,0% FFA) isoliert werden.

Der auf diese Weise gewonnene Feststoff weist einen höheren Anteil an Verunreinigungen (HMF und FFA) im Vergleich zum Feststoff aus Beispiel 1 auf.

### Literatur

Danielli, C., van Langen, L., Boes, D., Asaro, F., Anselmi, S., Provenza, F., Renzi, M., & Gardossi, L. (2022). 2,5-Furandicarboxaldehyde as a bio-based crosslinking agent replacing glutaraldehyde for covalent enzyme immobilization. RSC Advances, 12(55), 35676. https://doi.org/10.1039/D2RA07153C
Dai, J. (2021). Synthesis of 2,5-diformylfuran from renewable carbohydrates and its applications: A review. Green Energy & Environment, 6(1), 22-32. https://doi.org/10.1016/j.gee.2020.06.013
Acosta, A. M., Turull, C. C., Cosovanu, D., Marti, N. S., & Canela-Garayoa, R. (2021). Novel and Efficient Biotechnological Approach to Produce 2,5-Diformylfuran from Biomass-Derived 5-Hydroxymethylfurfural. ACS Sustainable Chemistry & Engineering, 9(43), 14550-14558. https://doi.org/10.1021/acssuschemeng.1c05308
Derflinger, C., Kamm, B., & Paulik, C. (2021). Sustainable aerogels derived from bio-based 2,5-diformylfuran and depolymerization products of lignin. International Journal of Biobased Plastics, 3(1), 29-39. https://doi.org/10.1080/24759651.2021.1877025
Cong, H., Yuan, H., Tao, Z., Bao, H., Zhang, Z., Jiang, Y., Huang, D., Liu, H., & Wang, T. (2021). Recent Advances in Catalytic Conversion of Biomass to 2,5-Furandicarboxylic Acid. Catalysts, 11(9), 1113. https://doi.org/10.3390/catal11091113
Amarasekara, A. S., Williams, L. D., & Ebede, C. C. (2008). Mechanism of the dehydration of D-fructose to 5-hydroxymethylfurfural in dimethyl sulfoxide at 150 °C: an NMR study. Carbohydrate Research, 343(18), 3021-3024. https://doi.org/10.1016/j.carres.2008.09.008
Laugel, C., Estrine, B., Le Bras, J., Hoffmann, N., Marinkovic, S., & Muzart, J. (2014). NaBr/DMSO-Induced Synthesis of 2,5-Diformylfuran from Fructose or 5-(Hydroxymethyl)furfural. ChemCatChem, 6(5), 1195-1198. https://doi.org/10.1002/cctc.201400023
Halliday, G. A., Young, R. J., & Grushin, V. V. (2003). One-Pot, Two-Step, Practical Catalytic Synthesis of 2,5-Diformylfuran from Fructose. Organic Letters, 5(11), 2003-2005. https://doi.org/10.1021/ol034572a
Li, Q., Ma, C.-L., & He, Y.-C. (2023). Effective one-pot chemoenzymatic cascade catalysis of biobased feedstock for synthesizing 2,5-diformylfuran in a sustainable reaction system. Bioresource Technology, 378, 128965. https://doi.org/10.1016/j.biortech.2023.128965
Qin, Y.-Z., Li, Y.-M., Zong, M.-H., Wu, H., & Li, N. (2015). Enzyme-catalyzed selective oxidation of 5-hydroxymethylfurfural (HMF) and separation of HMF and 2,5-diformylfuran using deep eutectic solvents. Green Chemistry, 17(7), 3718-3722. https://doi.org/10.1039/C5GC00788G
Tjallinks, G., Boverio, A., Jager, A. W., Kaya, S. G., Mattevi, A., & Fraaije, M. W. (2023). Efficient Oxidation of 5-Hydroxymethylfurfural Using a Flavoprotein Oxidase from the Honeybee Apis mellifera. ChemBioChem, 24(24), e202300588. https://doi.org/10.1002/cbic.202300588
Cajnko, M. M., Novak, U., Grilc, M., & Likozar, B. (2020). Enzymatic conversion reactions of 5-hydroxymethylfurfural (HMF) to bio-based 2,5-diformylfuran (DFF) and 2,5-furandicarboxylic acid (FDCA) with air: mechanisms, pathways and synthesis selectivity. Biotechnology for Biofuels, 13, 66. https://doi.org/10.1186/s13068-020-01705-z
McKenna, S. M., Mines, P., Law, P., Kovacs-Schreiner, K., Birmingham, W. R., Turner, N. J., Leimkühler, S., & Carnell, A. J. (2017). The continuous oxidation of HMF to FDCA and the immobilisation and stabilisation of periplasmic aldehyde oxidase (PaoABC). Green Chemistry, 19, 4660-4665. https://doi.org/10.1039/C7GC01696D
Milić, M., Byström, E., Dominguez de Maria, P., & Kara, S. (2024). Selective Oxidation of 5-Hydroxymethylfurfural to 2,5-Diformylfuran in Biphasic Media using Immobilized Galactose Oxidase: Proof of Concept and Limitations. ChemCatChem, 16(4), e202301384. https://doi.org/10.1002/cctc.202301384
Birmingham, W. R., Toftgaard Pedersen, A., Dias Gomes, M., Bøje Madsen, M., Breuer, M., Woodley, J. M., & Turner, N. J. (2021). Toward scalable biocatalytic conversion of 5-hydroxymethylfurfural by galactose oxidase using coordinated reaction and enzyme engineering. Nature Communications, 12, 4946. https://doi.org/10.1038/s41467-021-25034-3
Lin, B., & Tao, Y. (2017). Whole-cell biocatalysts by design. Microbial Cell Factories, 16, 106. https://doi.org/10.1186/s12934-017-0724-7
Protein [Internet]. Bethesda (MD): National Library of Medicine (US), National Center for Biotechnology Information. Accession No. WP_024766380.1, GMC oxidoreductase [Pseudomonas nitroreducens]. Verfügbar unter: https://www.ncbi.nlm.nih.gov/protein/WP_024766380.1 (Zugriff am 29.04.2024)
Viñambres, M., Espada, M., Martinez, A. T., & Serrano, A. (2020). Screening and Evaluation of New Hydroxymethylfurfural Oxidases for Furandicarboxylic Acid Production. Applied and Environmental Microbiology, 86(16), e00842-20. https://doi.org/10.1128/AEM.00842-20

## Patentansprüche

1. Verfahren zur Herstellung von 2,5-Diformylfuran, indem 5-(Hydroxymethyl)furfural, welches in einer einphasigen wässerigen Lösung gelöst vorliegt, mit einer Oxidase *in vitro* zu 2,5-Diformylfuran oxidiert wird, welches und aus der wässerigen Lösung abgetrennt wird, **dadurch gekennzeichnet, dass** das Verfahren bei einer Temperatur zwischen 20 °C und 40 °C ausgeführt wird und dass die einphasige wässerige Lösung zu Beginn der Oxidation mindestens 35 g/l 5-(Hydroxymethyl)furfural gelöst enthält, sodass das sich bildende 2,5-Diformylfuran unmittelbar aus der wässerigen Lösung ausfällt.

2. Verfahren nach Anspruch 1, **dadurch gekennzeichnet, dass** die einphasige wässerige Lösung das 5-(Hydroxymethyl)furfural zwischen 50 g/l und 250 g/l gelöst enthält.

3. Verfahren nach Anspruch 2, **dadurch gekennzeichnet, dass** die einphasige wässerige Lösung das 5-(Hydroxymethyl)furfural zwischen 150 g/l und 250 g/l gelöst enthält.

4. Verfahren nach einem der Ansprüche 1 bis 3, **dadurch gekennzeichnet, dass** es bei einer Temperatur zwischen 25 °C und 40 °C vorgenommen wird.
